# EUROPEAN PATENT APPLICATION

(11) **EP 3 520 638 A1**
(43) Date of publication of application: **07.08.2019**
(21) Application number: 17858342.3
(22) Date of filing: 02.10.2017
(51) Int. Cl.: A41D 1/06

(54) **UNDERGARMENT**

(30) Priority: 03.10.2016 JP 2016195517
(71) Applicant: Toray Industries, Inc., Tokyo 103-8666 (JP)
(72) Inventor: KASABO, Miki, Otsu-shi Shiga 520-8558 (JP); TAKADA, Nana, Otsu-shi Shiga 520-2141 (JP); MIYAMURA, Takako, Tokyo 101-0032 (JP)
(74) Representative: Kador & Partner PartG mbB
(86) International application number: PCT/JP2017/035807
(87) International publication number: WO 2018/066504

(57) **Abstract**

The purpose of the present invention is to provide a bottom wear capable of suppressing a situation in which an underwear worn by a wearer is slid in a wearer's stature direction with respect to the wearer's waist portion. The means for achieving the purpose is a bottom wear including a waist-encircling section, wherein
a cylindrical part including a cloth of bottom wear and a planar material different from the cloth, or a cylindrical part including a planar material different from the cloth of bottom wear is present at a back portion of the waist-encircling section and inside the bottom wear,
the cylindrical part is disposed in such a manner that the axis direction of the cylindrical part is substantially parallel to the waist-encircling direction of the waist-encircling section,
the static friction coefficient of the inner surface of a rear portion of the cylindrical part is 0.2 or more and 1.0 or less, and
the stress of a front portion of the cylindrical part at 10% elongation in the transverse direction of the cylindrical part is 10 N/25 mm or less.

## Description

### TECHNICAL FIELD

The present invention relates to a bottom wear.

### BACKGROUND ART

Among occupational diseases, low back pain is one of diseases incident to workers during working, and is spreading in various types of occupations. Examples of occupational workers who have a high risk of being attacked by low back pain include those relating to transportation business where workers are engaged in handling heavy weight articles and in long-distance driving and medical workers working for assistance of inpatients and treating emergency patients, and recently, the number of workers relating to services in social welfare facilities for care of the elderly and the like and suffering from low back pain has considerably increased. Further in recent years, personal computers have been spreading into job sites and homes, and a work for operating a mouse and a keyboard for many hours while gazing a display is a new factor of causing low back pain.

The number of occupational workers suffering from low back pain tends to increase as an age becomes older, but only a few people take a measure at an initial stage of low back pain, and there are not a few people who take no step until the low back pain gets worse. In addition, Japan is facing a problem with a decreased working-age population due to low birthrate and longevity, and there is an increasing number of senior workers of 65 years old or older and woman workers in the light of support of a society. Such being the case, importance of maintenance and promotion of health of valuable workers is increasing more than before, and also in case of working uniforms, a viewpoint of keeping health of workers positively becomes important in addition to conventional functions such as safety, easy action and discrimination.

In order to prevent low back pain, it has been considered effective to tighten a waist of a wearer, in particular a portion including an upper part of a pelvis of the wearer with a protection band for lumbago, and increase an abdominal pressure. The purpose of this is to decrease a burden of a waist by forming an abdominal cavity into a shape like a rugby ball to work as a support. However, there is a problem that since such a protection band for lumbago is mostly worn under a wear, and must be worn on a body after taking off the wear, fitting thereof is troublesome and that also when adjusting the fastening of the waist protection band after wearing it, since a degree of the fastening must be adjusted after the wear is taken off or loosened, this work is troublesome.

Thus, a sportswear with protections for lumbago is known in which protections for lumbago can be integrally attached to a sportswear such as sports trousers or the like, and the degree of tightening the protections for lumbago tightness can be easily adjusted (see Patent Document 1). This wear has a configuration in which it is possible to tighten a waist portion of a wearer by protections for lumbago and adjust the degree of tightening without taking off or loosening the wear, and the problem is solved that operations of tightening a waist portion of a wearer by protections for lumbago, etc. are troublesome.

### PRIOR ART DOCUMENT

### PATENT DOCUMENT

Patent Document 1: Japanese Patent Laid-open Publication No. 10-46409

### SUMMARY OF THE INVENTION

### PROBLEMS TO BE SOLVED BY THE INVENTION

However, in the sportswear with protections for lumbago as disclosed in Patent Document 1, the protections for lumbago are fixed around the waist of a wear main body. Thus, when a wearer repeatedly makes an expansion and contraction motion of changing the posture from an upright posture to a crouching posture or from a crouching posture to an upright posture over a plurality of times, the waist portion protecting portion may be slid in a wearer's stature direction with respect to a waist portion of the wearer as a portion around the waist of the main wear body is slid in the wearer's stature direction. The sportswear with protections for lumbago as disclosed in Patent Document 1 has a structure in which the protections for lumbago are in direct contact with an underwear worn by the wearer. Thus, the sportswear with protections for lumbago has the problem that when the upper end of the protections for lumbago is unintentionally locked in the underwear worn by the wearer, the underwear is slid in the wearer's stature direction with respect to the waist portion of the wearer as the protections for lumbago are slid in the wearer's stature direction with respect to the waist portion of the wearer, and thus the wearer has an unpleasant feeling.

Thus, the present invention has been made in view of the above-described problems, and an object of the present invention is to provide a bottom wear suitable for use with a protection band for lumbago for tightening a waist portion of a wearer, the bottom wear being capable of suppressing a situation in which an underwear worn by a wearer is slid in a wearer's stature direction with respect to the wearer's waist portion as the posture of the wearer tightened at the waist portion by the protection band for lumbago is changed as described above.

### SOLUTIONS TO THE PROBLEMS

For solving the above-described problems, the present invention discloses the following constitution and more preferable aspects.
1. A bottom wear including a waist-encircling section, wherein
   a cylindrical part including a cloth of bottom wear and a planar material different from the cloth, or a cylindrical part including a planar material different from the cloth of bottom wear is present at a back portion of the waist-encircling section and inside the bottom wear,
   the cylindrical part is disposed in such a manner that the axis direction of the cylindrical part is substantially parallel to the waist-encircling direction of the waist-encircling section,
   the static friction coefficient of the inner surface of a rear portion of the cylindrical part is 0.2 to 1.0, and
   the stress of a front portion of the cylindrical part at 10% elongation in the transverse direction of the cylindrical part is 10 N/25 mm or less.
2. The bottom wear according to item 1, wherein the static friction coefficient of the surface of a part of the front portion of the cylindrical part, which is close to a human body, is 0.2 to 1.0.
3. The bottom wear according to either of the above items, wherein the cylindrical part includes a cloth of bottom wear and a planar material different from the cloth, and
   the upper part and the lower part of the planar material are each continuously or intermittently connected to the cloth of bottom wear.
4. The bottom wear according to either of the above items, wherein the cylindrical part includes a planar material different from the cloth of bottom wear, and
   the upper part and the lower part of the cylindrical part are each continuously or intermittently connected to the cloth of bottom wear.
5. The bottom wear according to either of the above items, wherein the planar material is a fabric.
6. A bottom wear with a band which includes the bottom wear set forth in either of the above items, and a protection band for lumbago.

### EFFECT OF THE INVENTION

It is possible to provide a bottom wear suitable for use with a protection band for lumbago for tightening a waist portion of a wearer, the bottom wear being capable of suppressing a situation in which an underwear worn by a wearer is slid in a wearer's stature direction (hereinafter, sometimes referred to as "slipping-up") as is seen when the wearer repeatedly makes a motion of changing the posture from an upright posture to a crouching posture or from a crouching posture to an upright posture over a plurality of times.

### BRIEF DESCRIPTION OF THE DRAWINGS

Fig. 1 is a view of a bottom wear according to a first embodiment of the present invention where the bottom wear is seen from the front and above.
Fig. 2 is an external view of the bottom wear shown in Fig. 1.
Fig. 3 is a sectional view taken along line A-A' of the bottom wear shown in Fig. 1.
Fig. 4 is a front view of one example of a protection band for lumbago which can be attached to the bottom wear of the present invention.
Fig. 5 is a rear view of one example of a protection band for lumbago which can be attached to the bottom wear of the present invention.
Fig. 6 is a view of the bottom wear shown in Fig. 1, to which a protection band for lumbago is attached, where the bottom wear is seen from the front and above.
Fig. 7 is a sectional view taken along line B-B' of the bottom wear shown in Fig. 6, to which a protection band for lumbago is attached.
Fig. 8 is a view of a bottom wear according to a second embodiment of the present invention where the bottom wear is seen from the front and above.
Fig. 9 is a conceptual view of a vertical cross-section taken along line C-C' of the bottom wear shown in Fig. 8.
Fig. 10 is a view of the bottom wear shown in Fig. 8, to which a protection band for lumbago is attached, where the bottom wear is seen from the front and above.
Fig. 11 is a sectional view taken along line D-D' of the bottom wear shown in Fig. 10, to which a protection band for lumbago is attached.
Fig. 12 is a sectional view taken along line E-E' of the bottom wear shown in Fig. 2.
Fig. 13 is a right side view of the bottom wear shown in Fig. 1.
Fig. 14 is a right side view of the bottom wear shown in Fig. 8.

### EMBODIMENTS OF THE INVENTION

The meanings of the terms used in the present invention are as follows
Bottom wear: a wear worn on a lower body, e.g. a skirt or pants such as slacks, jeans, chino pants, cargo pants and shorts.

Waist-encircling section: a part or the whole of a bottom wear which comes into direct or indirect contact with a portion around a body surface at a portion ranging from a lower part of a spinal column to a pelvis of a wearer at the time when the bottom wear is worn. The waist-encircling section may have at least one selected from the group consisting of a waist portion including a belt loop, side pockets, back pockets and front and back fasteners, and a rise portion of trousers.

Back portion: a part of a bottom wear which comes into direct or indirect contact with the back of a wearer.

Cylindrical part: a cylindrical part present on a waist-encircling section and a back portion of a bottom wear. The cylindrical part is present on the front part of the back portion of the bottom wear main body. The cylindrical part is not necessarily required to have a circular cross-section, and may have a hollow cross-section, or a cross-section which can be made hollow. The cylindrical part may be collapsed, or folded into a substantially planar shape. The axial direction of the cylindrical part is also a length direction of the cylindrical part.

Cylindrical part in the first embodiment of the present invention: a cylindrical part including a cloth of bottom wear and a planar material different from the cloth.

Cylindrical part in the second embodiment of the present invention: a cylindrical part including a planar material different from a cloth of bottom wear.

Invention of the first embodiment: an invention having a cylindrical part according to the first embodiment.

Invention of the second embodiment: an invention having a cylindrical part according to the second embodiment.

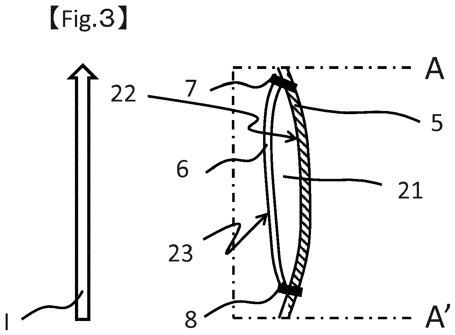

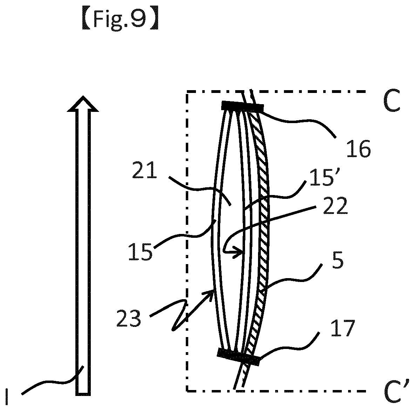

Rear portion of cylindrical part: the cylindrical part is connected to the bottom wear at the upper part of the rear portion. The rear portion can be divided into two portions by a connecting line and a line with the cylindrical part situated on the lowermost side. Among the above-mentioned portions, the rear portion is a portion far from a wearer. In the first embodiment, the rear portion is a "bottom wear cloth 5 on a back portion in a waist-encircling section" in Fig. 3. In the second embodiment, the rear portion is a "rear portion 15' of a cylindrical part" in Fig. 9. Hereinafter, it is sometimes referred to as a "rear portion of a cylindrical part".

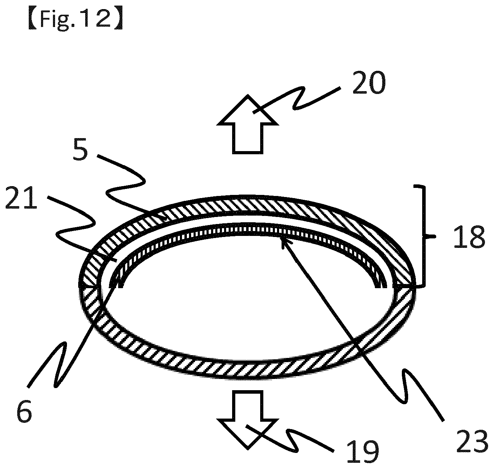

Front portion of cylindrical part: the cylindrical part is connected to the bottom wear at the upper part of the rear portion. The rear portion can be divided into two portions by a connecting line and a line with the cylindrical part situated on the lowermost side, and among the above-mentioned portions, the front portion is a portion close to a wearer. In the first embodiment, the front portion is a "front portion 6 of a cylindrical part" in Fig. 3. In the second embodiment, the front portion is a "front portion 15 of a cylindrical part" in Fig. 9. Hereinafter, it is sometimes referred to as a "front portion of a cylindrical part".
Front: a frontward direction of a wearer in a bottom wear (symbol 19 in Fig. 12).
Rear: a rearward direction of a wearer in a bottom wear (symbol 20 in Fig. 12).

Hereinafter, the present invention will be described with reference to the drawings, but the present invention is not limited to the following disclosure.

First, the bottom wear according to the first embodiment will be described.

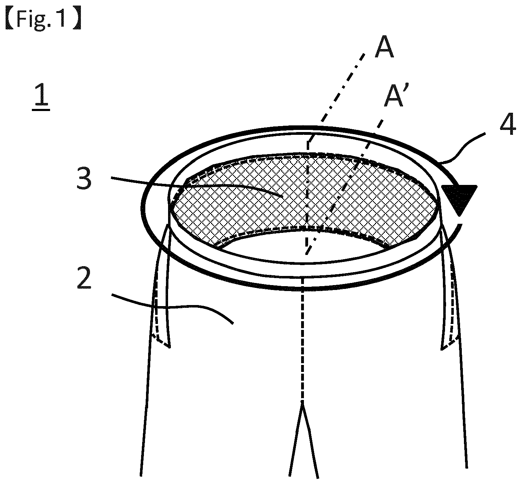

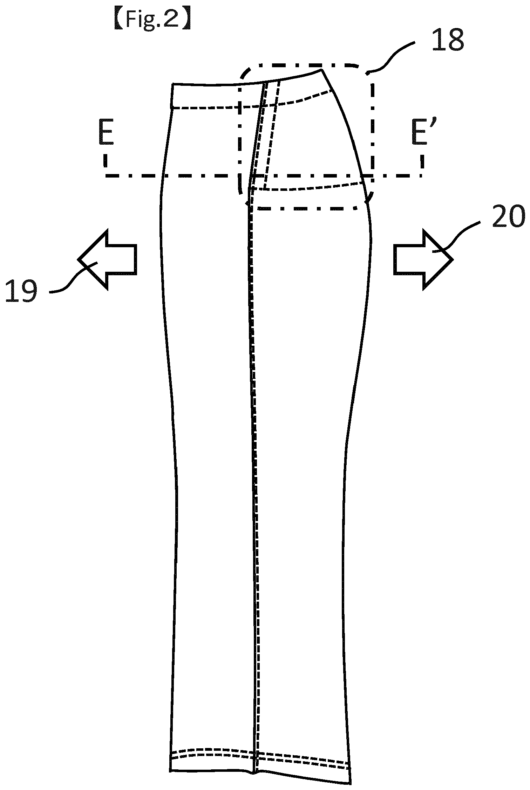

Fig. 1 is a view showing a part of the bottom wear as an example of the first embodiment, where the part of the bottom wear is seen from the front and above, and Fig. 2 is a right side view of the bottom wear shown in Fig. 1. The bottom wear 1 of the present invention has a waist-encircling section 2 around the bottom wear.

In addition, the waist-encircling section 2 of the bottom wear 1 according to the first embodiment has a cylindrical part 3 on a back portion 18 thereof. The cylindrical part 3 is disposed in such a manner that the axis direction of the cylindrical part is substantially parallel to a waist-encircling direction 4 of the waist-encircling section.

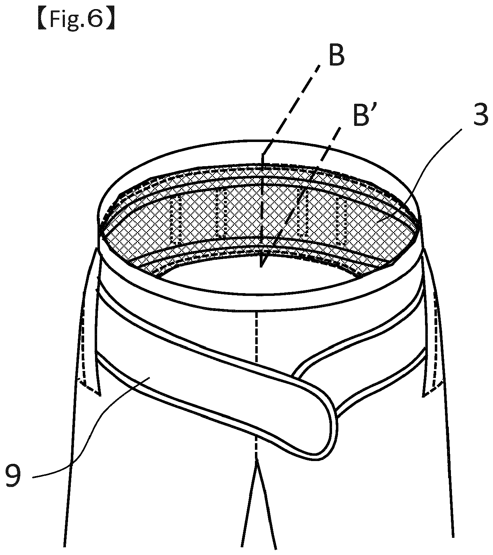

See Fig. 6. Inside the cylindrical part 3, a protection band for lumbago 9 can be inserted into the cylindrical part 3.

Next, Fig. 3 is a sectional view taken along line A-A' of the bottom wear shown in Fig. 1. As shown in Fig. 3, the cylindrical part 3 in Fig. 1 includes a bottom wear cloth 5 on a back portion in the waist-encircling section, and a planar material forming a cylindrical part front portion 6, and the planar material is disposed inside the bottom wear. The bottom wear cloth 5 is a cylindrical part rear portion of the cylindrical portion in the present invention. In addition, a planar material is used for the cylindrical part front portion 6. As a result, a cylindrical part including a cloth of bottom wear and a planar material different from the cloth is formed.

The upper end of the cylindrical part front portion 6 including the planar material is continuously or intermittently connected to the bottom wear cloth 5 by a locking portion 7 in the waist-encircling direction. Preferably, the upper end of the planar material forming the cylindrical part front portion 6 is connected to the waist-encircling section as in the configuration in Fig. 3 because it is possible to suppress a situation in which the toe or the like of a wearer is caught between the planar material and the bottom wear cloth 5 at the time of wearing the bottom wear of the present invention.

It is to be noted that the present invention is not limited to the configuration shown in Fig. 3. A portion slightly below the upper end of the planar material (a part situated below the upper end of the planar material) may be connected, i.e. the upper end of the planar material may be protruded above the locking portion.

In addition, in Fig. 3, the lower end of the planar material forming the cylindrical part front portion 6 is intermittently connected in a waist-encircling direction to the bottom wear cloth 5 on the back portion in the waist-encircling section by a locking portion 8. The reason why it is preferable that the planar material is connected at the lower end is as follows.

The lower end portion of the planar material can be fixed so as to extend along the inner surface of the cloth of bottom wear. As a result, a waist portion band is neatly inserted into the cylindrical part. In addition, it is possible to suppress uncontrollable movement of the lower end portion of the planar material inside the bottom wear. It is possible to suppress bending of the planar material inside the bottom wear, and therefore a wearer can smoothly insert a protection band for lumbago into the cylindrical part while wearing the bottom wear.

The present invention is not limited to the configuration shown in Fig. 3. A portion slightly above the lower end of the planar material (a part situated above the lower end of the planar material) may be connected, i.e. the lower end of a fabric may be protruded below the locking portion.

In the bottom wear configured to have a cross-section as in Fig. 3 in the present invention, the locking portion 7 and the locking portion 8 are formed by stitch, and the upper part and the lower part of the fabric (planar material) are each intermittently connected in the waist-encircling direction to the bottom wear cloth on the back portion in the waist-encircling section. The connection is not required to be intermittent, and may be continuous. Preferably, the connection is performed at the upper end and the lower end of the planar material where a cylindrical body is formed. Here, examples of the intermittent locking portion include those obtained by stitch, an adhesive, welding, or locking with a hook-and-loop fastener or button. On the other hand, examples of the continuous locking portion include those obtained by stitch or welding. The stitch may be normal lock stitch, but is more preferably chain stitch such as flat seam stitch, two needles stitch or three needles stitch from the viewpoint of smoothing the texture to a person. In addition, an example of the configuration of the intermittent locking portion is not limited to the configuration of the locking portion of the bottom wear of the present invention shown in Fig. 1, and may be, for example, a configuration in which only one locking portion is provided at the lower end of both end portions of the cylindrical part in the axis direction of the cylindrical part.

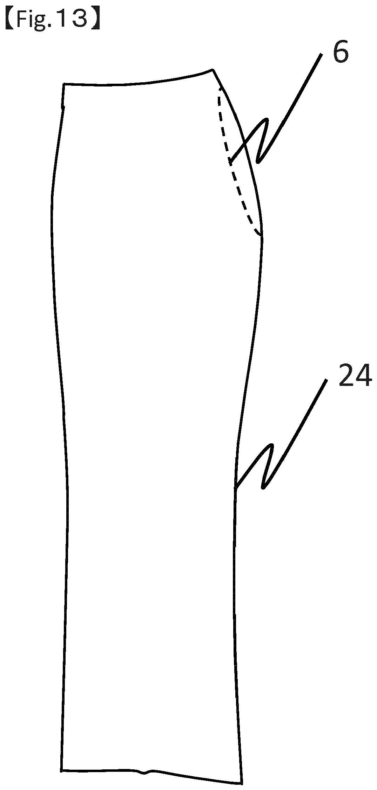

The cloth of bottom wear in the bottom wear according to the first embodiment will now be described. In the bottom wear of this embodiment, the cloth of bottom wear is a cloth other than the planar material forming the cylindrical part front portion, among members forming the bottom wear. Fig. 13 is an external view of the bottom wear shown in Fig. 1. In Fig. 13, a planar material forming the cylindrical part front portion 6 is shown by a broken line, which is an invisible part, and a cloth of bottom wear 24 is shown by a solid line.

Fig. 12 is a sectional view taken along line E-E' of the bottom wear shown in Fig. 2. In the embodiment of the bottom wear of the present invention shown in Fig. 1, a cylindrical part including the bottom wear cloth 5 on the back portion in the waist-encircling section, and a planar material forming the cylindrical part front portion 6 is formed in a back portion 18 in the waist-encircling section. In addition, a space in the cylindrical part, i.e. a space between the bottom wear cloth 5 on the back portion in the waist-encircling section and the cylindrical part front portion 6 is a space 21 for placing a protection band for lumbago, and in a state of a bottom wear with a band, to which a band is attached, a waist portion protecting portion of the protection band for lumbago is stored in the space 21 for placing a protection band for lumbago.

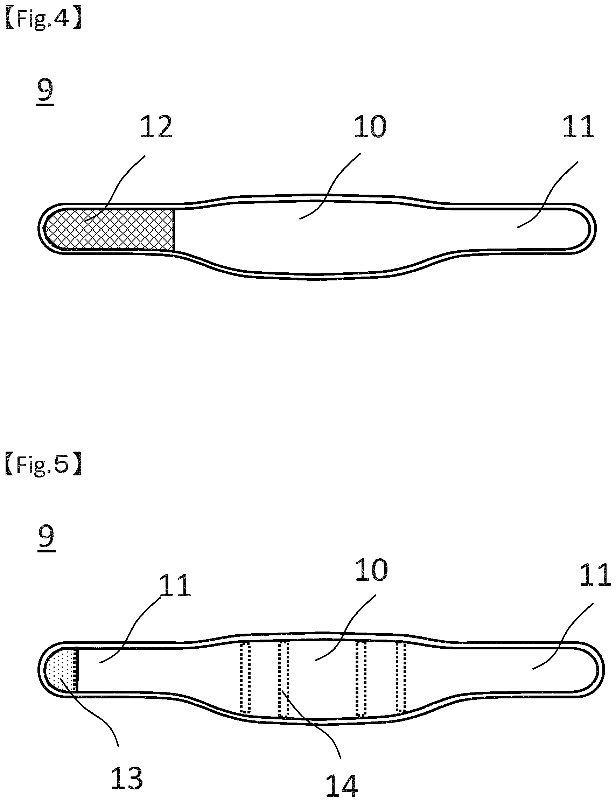

Next, Fig. 4 is a front view showing one surface of one example of a protection band for lumbago which can be attached to any of the bottom wears of the first and second embodiments. Fig. 5 is a rear view of the protection band for lumbago. The protection band for lumbago 9 includes a waist portion protecting portion 10, a belt portion 11 disposed on each of the left and the right of the waist portion protecting portion 10, a first fixing member 12 disposed at one end portion of the protection band for lumbago, a second fixing member 13 disposed at the other end portion of the protection band for lumbago, and a bone 14 disposed at the center of the waist portion protecting portion 10.

The waist portion protecting portion 10 of the protection band for lumbago 9 shown in Figs. 4 and 5 is in the form of a band having a large width. The large width is suitable for disposing the bone. Unlike the illustrated waist portion protecting portion, the waist portion protecting portion may have a width equal to or smaller than the width of the belt portion. In addition, the width of the waist portion protecting portion in a stature direction of a wearer is preferably 5 to 20 cm. When the back width is 5 cm or more, a fastening pressure on a waist portion of the wearer is concentrated on a narrow width portion and becomes relatively high, thereby enabling a blood vessel to be inhibited from being excessively compressed, and further, in positioning of the waist portion protecting portion, a burden for positioning the waist portion protecting portion on an upper part of a pelvis can be reduced. When the width is 20 cm or less, oppressive heat can be reduced at the time of wearing the bottom wear for many hours. In consideration of these points mentioned above, in order to allow the bottom wear to be used for many hours and secure workability while minimizing a limitation in a range of motion, it is preferable to set the width of the waist portion protecting portion to be within the mentioned range. In addition, from the viewpoint mentioned above, it is more preferable that the width is 8 cm or more, and the width is 15 cm or less.

The waist portion protecting portion may be made of a material being the same as or different from that of the belt portion. Fabric, synthetic leather or the like can be used as the material of the waist portion protecting portion and the belt portion. The material is preferably fabric because the protection band for lumbago can be made selectable. As the material of the above-mentioned fabric, synthetic fibers such as polyethylene terephthalate fiber and polyamide fiber, natural fibers such as cotton fiber, regenerated fibers such as rayon fiber, and the like can be used. In addition, as the material of the above-mentioned fabric, a plurality of the above-described fibers can be used.

Further, it is preferable that the waist portion protecting portion is provided with bones for stabilizing a position of a spinal column. Here, the material of the bone is preferably a metal, a resin or the like which has a relatively high bending stress. Examples of the shape include a spiral shape and a strip shape.

In addition, the waist portion protecting portion may be provided with a slip prevention member (not shown).

When in a state in which the protection band for lumbago is attached to the bottom wear of the present invention, the first fixing member and the second fixing member of the protection band for lumbago are joined together in front of the wearer, the pressure on the waist portion of the wearer can be increased. It is preferable that at least one of the first fixing member and the second fixing member is long in the longitudinal direction of the protection band for lumbago because the adjustable range of the pressure on the waist portion of the wearer can be expanded. Further, for the purpose of expanding the adjustable range of the pressure on the waist of the wearer, an auxiliary belt composed of band-shaped flat rubber or the like may be provided.

Here, it is preferable to use a hook-and-loop fastener as a material for the first fixing member and the second fixing member of the protection band for lumbago so that the first fixing member and the second fixing member are mutually locked. In this case, the type of hook-and-loop fastener is not particularly limited, but when considering that pilling occurs in the cloth of bottom wear due to damage to the cloth of bottom wear, it is preferable to use a hook-and-loop fastener of mushroom type in which the tip portion of a protrusion portion of a male surface is rounded.

Further, when the male surface and the female surface of the hook-and-loop fastener used for the protection band for lumbago are locked, the shear strength is preferably 4.0 N/cm² or more, more preferably 8.0 N/cm² or more for preventing detachment of the locking portion of the protection band for lumbago at the time of wearing the protection band for lumbago. On the other hand, the shear strength is preferably 12.0 N/cm² or less for maintaining comfort at the time of taking on or taking off the protection band for lumbago. When a hook-and-loop fastener having a shearing strength in the above-mentioned range is used for the protection band for lumbago, the force with which the two overlapping end portions attract each other is moderate, so that further excellent waist portion wearability is exhibited.

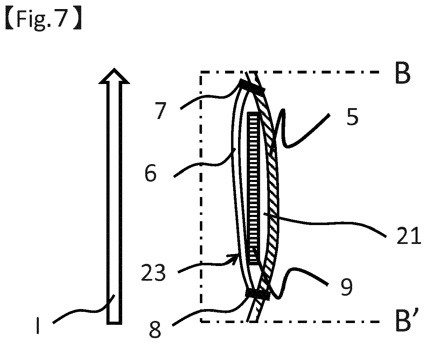

Fig. 6 is a view showing a state in which the protection band for lumbago shown in Fig. 4 is attached to the bottom wear in Fig. 1, where the bottom wear is seen from the front and above. Fig. 7 is a sectional view taken along line B-B' of the bottom wear and protection band for lumbago attached thereto in Fig. 6. In this embodiment, the protection band for lumbago 9 is inserted and disposed inside the cylindrical part 3 in such a manner that the longitudinal direction of the protection band for lumbago 9 is substantially parallel to the axis direction of the cylindrical part 3, and a surface of the protection band for lumbago, which is provided with a bone (hereinafter, the surface of the protection band for lumbago is sometimes referred to as a "surface A of protection band for lumbago"), is on the wearer side at the time of wearing the bottom wear. The right end portion of the protection band for lumbago is protruded from the cylindrical part outside the cylindrical part on the right side of the cylindrical part 3 as shown in Fig. 6. The left end portion of the protection band for lumbago is protruded from the cylindrical part outside the cylindrical part on the left side of the cylindrical part 3. In addition, the waist portion protecting portion of the protection band for lumbago is covered with the cylindrical part, and the inner surface of the cylindrical part rear portion is in contact with a surface on the side opposite to the surface A of protection band for lumbago (hereinafter, the surface of the protection band for lumbago is sometimes referred to as a "surface B of protection band for lumbago"). That is, the surface B of protection band for lumbago is in contact with the inner surface of the bottom wear cloth on the back portion in the waist-encircling section of the cloth of bottom wear.

See Fig. 3. In the bottom wear of the first embodiment, the bottom wear cloth 5 on the back portion in the waist-encircling section corresponds to the cylindrical part rear portion. Thus, the static friction coefficient of the inner surface 22 is 0.2 to 1.0.

In addition, the planar material forming the cylindrical part front portion 6 in Fig. 3 has a stress of 10 N/25 mm or less at 10% elongation in a transverse direction (hereinafter, sometimes referred to as a direction I). Here, the direction I is also a direction substantially parallel to the wearer's stature height direction at the time of wearing the bottom wear.

Here, the material of the planar material is not particularly limited, but a fabric is preferable because it is excellent in versatility. In particular, a woven fabric or a knitted fabric is more preferable. Examples of the fiber used for the fabric may include synthetic fibers and natural fibers, and a plurality of fibers may be used. When considering washing resistance and versatility of original yarn/weaving and knitting design, it is preferable that synthetic fiber such as polyester fiber or nylon fiber is used alone, or synthetic fiber such as polyester fiber or nylon fiber and at least one selected from the group consisting of polyurethane fiber, rayon fiber, acrylic fiber and cotton fiber are used in combination.

As described above, the planar material of the cylindrical part front portion (symbol 6 in Fig. 7) has a stress of 10 N/25 mm or less at 10% elongation in a transverse direction i.e. a direction I. A fabric that can be used for achieving the above-mentioned stress is a woven fabric using a stretchable yarn as each of warp and weft yarns or as one of warp and weft yarns, or a knitted fabric more extensible as compared to a woven fabric. The knitted fabric can be obtained by appropriately adjusting the blending ratio of a stretchable yarn and a non-stretchable yarn. Specific examples of the fabric having a stress of 10 N/25 mm or less at 10% elongation in the direction I include the following fabrics.
(I) Woven fabrics composed of fiber obtained by mixing elastic fiber (such as polyurethane fiber) with natural fiber such as silk or wool fiber, regenerated fiber such as rayon fiber, synthetic fiber such as acrylic fiber or polyester fibers, or the like.
(II) Knitted fabrics composed of fiber obtained by mixing elastic fiber (such as polyurethane fiber) with natural fiber such as silk or wool fiber, regenerated fiber such as rayon fiber, synthetic fiber such as acrylic fiber or polyester fibers, or the like.
(III) Woven fabrics in which elastic fiber is not used, but multifilaments of composite fiber obtained by bonding two polyester resins, one of which is a polyester resin mainly composed of polytrimethylene terephthalate (PTT) and the other of which is a polyester resin mainly composed of polyethylene terephthalate, side by side along the fiber length are used as at least one of a warp yarn and a weft yarn.
(IV) Knitted fabrics in which elastic fiber is not used, but multifilaments of composite fiber obtained by bonding two polyester resins, one of which is a polyester resin mainly composed of polytrimethylene terephthalate (PTT) and the other of which is a polyester resin mainly composed of polyethylene terephthalate, side by side along the fiber length are used.
(V) Power net.

Here, the power net refers to a fine net-like cloth produced from nylon fiber or the like and elastic fiber (e.g. polyurethane fiber). The power net may be a relatively thin cloth having an areal weight of about 100 g/m², and the cylindrical body front portion 6 (i.e. planar material) is preferably a power net because it is excellent in elongation and kickback performance, and capable of making the cylindrical part compact while securing the function of the cylindrical part provided on the back portion of the bottom wear.

As described above, the static friction coefficient of the inner surface 22 of the cylindrical part rear portion (the bottom wear cloth 5 on the back portion in the waist-encircling section in Fig. 7) is 0.2 or more and 1.0 or less. The reason why this static friction coefficient is selected is as described below. Here, examples of the means for setting the static friction coefficient of the inner surface 22 of the cylindrical part rear portion to 0.2 to 1.0 include the following.

The cloth of bottom wear is formed into a woven fabric or a knitted fabric, and it is possible to adjust the static friction coefficient according to the irregular state of the surface of this cloth and the kind of yarn forming the cloth. For the woven fabric, the static friction coefficient is small in the case of a plain weave having a small number of irregularities on the surface, and conversely, the static friction coefficient is large in the case of a twill weave in which oblique ridges appear on the woven fabric surface, and a satin weave in which oblique ridges do not appear, but tissue points are arranged at regular intervals. In addition, the static friction coefficient is generally increased by providing a pile on the surface of the cloth or raising the surface. For the knitted fabric, weft knitting or warp knitting may be performed, and the static friction coefficient can be adjusted according to the density of the knitted fabric. However, warp knitting is preferable because even when the stitch is cut, a run hardly occurs, resulting in enhancement of durability. The static friction coefficient can also be adjusted according to the shape of the yarn to be used for the woven or knitted fabric. For example, since the irregularities on the fiber surface of a filament yarn is smaller as compared to a spun yarn, the static friction coefficient of a cloth using the filament yarn can be reduced. In addition, the static friction coefficient can also be adjusted according to the material of the yarn. For example, polytetrafluoroethylene fiber (PTFE fiber) itself has a low static friction coefficient, and conversely, rubber or a polyurethane yarn itself has a high static friction coefficient. By using such a yarn for a part or the whole of the cloth, the static friction coefficient as a cloth can be adjusted. Further, the static friction coefficients of the surface and the back surface of the cloth can be set to different values. When on one surface of a double-side knitted structure (tricot), a knit loop is formed with only an elastic yarn to form a warp knitted fabric, and on the other surface, a knit loop is formed by use of an elastic yarn and an inelastic yarn in combination to form a warp knitted fabric, a high friction coefficient is developed for the surface on which the knit loop is formed with only an elastic yarn.

In addition, the static friction coefficient of an outer surface 23 of the cylindrical part front portion (i.e. a surface of the cylindrical part, which is close to the human body) is preferably 0.2 to 1.0. In addition, as a method for adjusting the static friction coefficient, the static friction coefficient is adjusted according to irregularities on the surface of the woven or knitted structure, and the shape and type of a yarn as in the method for adjusting the static friction coefficient of the inner surface of the bottom wear cloth 5 on the back portion in the waist-encircling section.

Since the static friction coefficient of the outer surface of the cylindrical part front portion is 0.2 to 1.0, the present invention more remarkably exhibits an effect of suppressing a situation in which an underwear worn by a wearer is slid in a wearer's stature direction with respect to a waist portion of the wearer as the wearer makes a motion.

Subsequently, a bottom wear having a cylindrical part according to the second embodiment will be described below.

The cylindrical part according to the second embodiment includes a planar material different from a cloth of bottom wear.

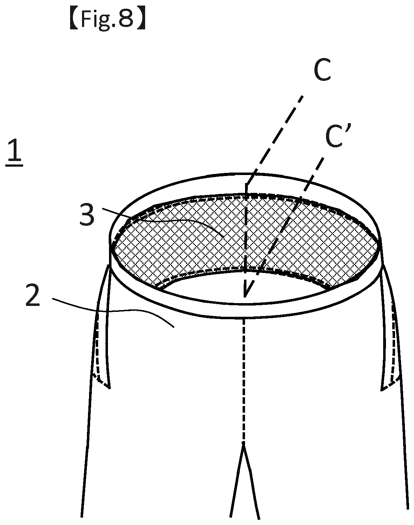

Fig. 8 is a view of the bottom wear according to the second embodiment where the bottom wear is seen from the front and above. Fig. 9 is a view of a vertical cross-section taken along line C-C' of the bottom wear shown in Fig. 8. In this embodiment, as shown in Fig. 8, the bottom wear 1 has a waist-encircling section 2, and a cylindrical part 3 on the back potion of the waist-encircling section 2. Further, the axis direction of the cylindrical part 3 is substantially parallel to a waist-encircling direction of the waist-encircling section. The cylindrical part according to the second embodiment includes a planar material different from the cloth of bottom wear, and the cylindrical part includes a cylindrical part front portion 15 and a cylindrical part rear portion 15' as shown in Fig. 9. The upper end and the lower end of the cylindrical part are connected to the bottom wear cloth on the back portion in the waist-encircling section of the cloth of bottom wear by a locking portion 16 and a locking portion 17, respectively.

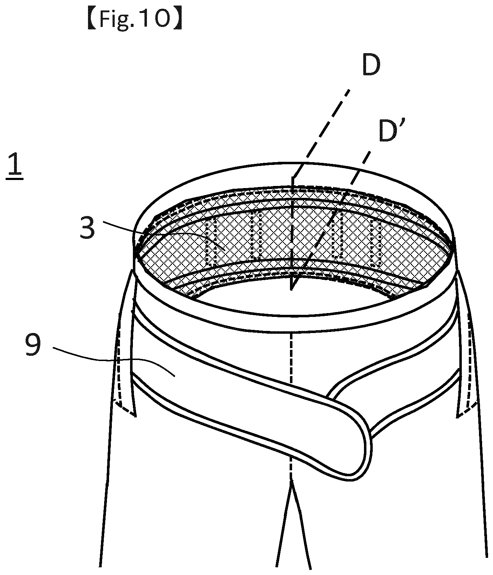

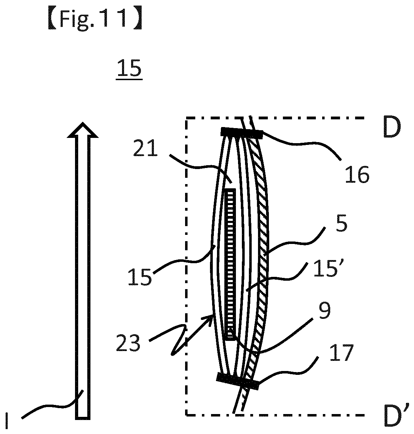

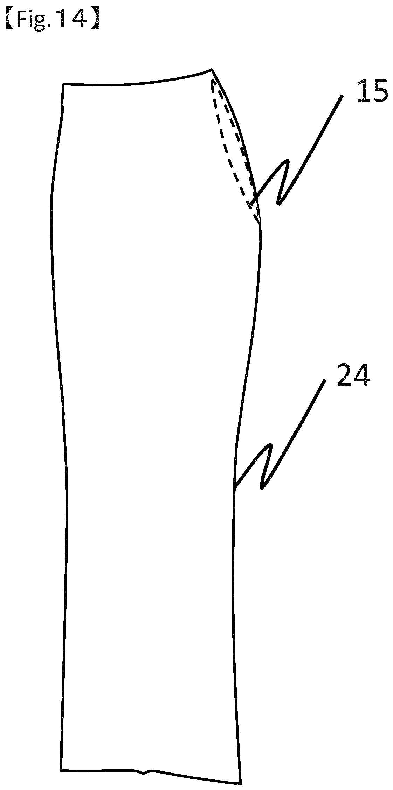

Fig. 14 is an appearance view of the bottom wear of the second embodiment as shown in Fig. 8. In Fig. 14, the cylindrical part is shown by a broken line, which is an invisible part, and the cloth of bottom wear 24 is shown by a solid line. Fig. 10 is a view showing a state in which the protection band for lumbago shown in Figs. 4 and 5 is attached to the bottom wear shown in Fig. 8 as the second embodiment, where the bottom wear is seen from the front and above. Fig. 11 is a sectional view taken along line D-D' of the bottom wear shown in Fig. 10, to which a protection band for lumbago is attached.

See Fig. 10. In the second embodiment, the protection band for lumbago 9 is inserted and disposed inside the cylindrical part 3. The longitudinal direction of the protection band for lumbago 9 is substantially parallel to the axis direction of the cylindrical part 3, and the surface A of the protection band for lumbago is on the wearer side at the time of wearing the bottom wear. The right end portion of the protection band for lumbago on the right side of the cylindrical part 3 is protruded from the cylindrical part, and the left end portion of the protection band for lumbago is protruded from the cylindrical part outside the cylindrical part on the left side of the cylindrical part. In addition, the waist portion protecting portion of the protection band for lumbago is covered with the cylindrical part, and the inner surface of the cylindrical part rear portion may be in contact with the surface B of the protection band for lumbago. That is, the surface B of protection band for lumbago may be in contact with a surface on a side opposite to a surface that is in contact with the inner surface of the bottom wear cloth on the back portion in the waist-encircling section of the cloth of bottom wear of a fabric 15 having a cylindrical shape.

In the bottom wear of the second embodiment, the static friction coefficient of the inner surface (surface denoted by symbol 22 in Fig. 9) of the cylindrical part rear portion is 0.2 to 1.0. The reason why this static friction coefficient is selected is as described below. The cylindrical part front portion has a stress of 10 N/25 mm or less at 10% elongation in a transverse direction (direction I).

In the second embodiment, the material of the planar material forming the cylindrical part is not particularly limited, but is preferably a fabric. As materials having a static friction coefficient as described above and a stress at elongation as described above, those described in the first embodiment can be used. Examples of the method for forming the cylindrical part include a method in which a fabric is folded, and the upper parts are connected; a method in which two or more fabrics are stitched together; and a method in which a circular knitted fabric is used as such to form a cylindrical shape. The material may be used as such to form the cylindrical part.

The cylindrical part formed by the cylindrical part front portion 15 and rear portion 15' is continuously or intermittently connected to the bottom wear cloth 5 at the upper end by the locking portion 16 in the waist-encircling direction. Preferably, the upper end of the cylindrical material is connected to the waist-encircling section because it is possible to suppress a situation in which the toe or the like of a wearer is caught between the cylindrical material and the bottom wear cloth 5 at the time of wearing the bottom wear of the present invention.

It is to be noted that the present invention is not limited to the configuration shown in Fig. 9. A portion slightly below the upper end of the planar material (a part situated below the upper end of the planar material) may be connected, i.e. the upper end of the planar material may be protruded above the locking portion.

In addition, in Fig. 9, the lower end of the cylindrical part is intermittently connected in a waist-encircling direction to the bottom wear cloth 5 on the back portion in the waist-encircling section by a locking portion 17. The reason why it is preferable that the planar material is connected at the lower end is as follows.

The lower end portion of the cylindrical part can be fixed so as to extend along the inner surface of the cloth of bottom wear. As a result, a waist portion band is neatly inserted into the cylindrical part. In addition, it is possible to suppress uncontrollable movement of the lower end portion of the cylindrical part inside the bottom wear. It is possible to suppress bending of the cylindrical part inside the bottom wear, and therefore a wearer can smoothly insert a protection band for lumbago into the cylindrical part while wearing the bottom wear.

The present invention is not limited to the configuration shown in Fig. 9. A portion slightly above the lower end of the planar material (a part situated above the lower end of the planar material) may be connected, i.e. the lower end of a fabric may be protruded below the locking portion.

In the bottom wear configured to have a cross-section as in Fig. 9 in the present invention, the cylindrical part is connected to the bottom wear cloth on the back portion by the locking portion 16 and the locking portion 17. However, the upper end or the lower end of the fabric may be continuously connected to the bottom wear cloth.

Here, examples of the intermittent or continuous locking portion include the means described in the first embodiment.

In addition, in the second embodiment as well as the first embodiment, the static friction coefficient of the outer surface of the cylindrical part front portion (i.e. a surface of the cylindrical part, which is close to the human body) is preferably 0.2 to 1.0. That is, in this embodiment, the static friction coefficient of a surface of the planar material forming the front portion of the cylindrical part, which is close to the human body, is preferably 0.2 or more and 1.0 or less. Here, the surface of the planar material forming the cylindrical part, which is close to the human body, is a surface of the cylindrical part front portion 15, which may be in contact with the body of the wearer or an underwear worn by the wearer, at the time of wearing the bottom wear shown in Figs. 10 and 11. The means for adjusting the static friction coefficient of the fabric is as described above. Since the static friction coefficient of the surface of the cylindrical part front portion, which is close to the human body, is 0.2 to 1.0, the present invention more remarkably exhibits an effect of suppressing a situation in which an underwear worn by a wearer is slid in a wearer's stature direction with respect to a waist portion of the wearer as the wearer makes a motion.

The effect of the bottom wear of the second embodiment as well as the bottom wear of the first embodiment in which the static friction coefficient and the stress at elongation are each a specific range will be described. The static friction coefficient of the inner surface of the cylindrical part rear portion of the waist-encircling section provided in the bottom wear is 0.2 to 1.0, and the stress of the cylindrical part front portion at 10% elongation is 10 N/25 mm or less. Since the bottom wear of the present invention has the above-mentioned characteristics, occurrence of a phenomenon is suppressed in which the underwear of the wearer is pulled upward as the wearer makes a motion such as bending and stretching. A supposed mechanism in which the above-described effect is exhibited may be as follows.

That is, when the wearer wears an underwear with a protection band for lumbago, and the wearer's waist portion is tightened with the protection band for lumbago, the protection band for lumbago firmly adheres to the waist portion of the wearer while the underwear worn by the wearer is interposed between the protection band for lumbago and the waist portion. In this state, the static friction coefficient of the inner surface of the cylindrical part rear portion of the waist-encircling section provided in the bottom wear of the present invention is as small as 0.2 or more and 1.0 or less, and therefore the protection band for lumbago moves upward while being moderately slid upward in the cylindrical part in conformity to upward movement of the waist-encircling section of the bottom wear with a motion of the wearer such as bending and stretching. The cylindrical part front portion moderately extends upward, and the protection band for lumbago conforms to the motion while maintaining an appropriate position, e.g. a position fit to the pelvis. When the wearer then returns from the stretching state to the standing posture, the belt moves to an original position while being moderately slid downward in the cylindrical part, and the cylindrical part front portion is shrunk to return to an original size. When the human body bends, the abdominal circumference becomes larger, leading to an increase in pressure on the protection band for lumbago. The point is the movement of the protection band for lumbago when the wearer returns from the bending state to the standing posture. When the human body returns from the bending state to the standing posture, the abdominal circumference becomes smaller from the large state. At that time, the human body is getting thinner as going from the waist portion to the waistline. When a bottom wear having no cylindrical part is worn after a protection band for lumbago is attached, the protection band for lumbago rises toward the waistline, and remains as it is.

However, when the protection band for lumbago extends through the cylindrical part of the bottom wear, and the static friction coefficient and stress of each portion of the cylindrical part is within the above-described range, the waist-encircling section of the bottom wear returns to an original position. In addition, the protection band for lumbago acts to return to the original position while being moderately pulled in the direction I and slid in the cylindrical part, the protection band for lumbago hardly rises and remains as it is.

In the second embodiment as well as the first embodiment, the static friction coefficient of the inner surface of the cylindrical part rear portion provided in the bottom wear of the present invention is 0.2 to 1.0 as described above. Since the static friction coefficient of the inner surface of the cylindrical part rear portion is 1.0 or less, it is possible to suppress a situation in which the protection band for lumbago moves in conformity to the movement of the waist-encircling section of the bottom wear. On the other hand, since the static friction coefficient of the inner surface of the cylindrical part rear portion is 0.2 or more, the following effect is exhibited. That is, it is possible to suppress a situation in which the protection band for lumbago excessively moves excessively independently of the waist-encircling section of the bottom wear, and the position of the protection band for lumbago is deviated from a position suitable for protection of the waist portion. The static friction coefficient is preferably 0.3 or more, more preferably 0.4 or more because the above-described effect can be further improved. In addition, the static friction coefficient is more preferably 0.8 or less, still more preferably 0.6 or less because the above-described effect can be further improved.

The static friction coefficient of a surface of a portion of the cylindrical part front portion, which is far from the human body (i.e. a surface of the cylindrical part front portion) on a side opposite to a surface on the wearer side) has substantially no influence on the effect of the present invention: i.e. suppressing a situation in which the underwear worn by the wearer is slid in the wearer's stature direction with respect to the waist portion of the wearer. The mechanism thereof may be as follows. That is, in order to obtain the effect of the present invention, it is necessary that the waist portion protecting portion of the protection band for lumbago and the cylindrical part rear portion be easily slid relatively in the wearer's stature direction as the wearer makes a bending-and-stretching motion. Here, when the wearer wears the bottom wear of the present invention and the protection band for lumbago, the cylindrical part front portion is pressed against the waist portion of the wearer by the waist portion protecting portion of the protection band for lumbago. Thus, even if the static friction coefficient of the surface of the cylindrical part front portion, which is far from the human body, and the waist portion protecting portion is low, the waist portion protecting portion of the protection band for lumbago is hardly slid with respect to the cylindrical part front portion because the cylindrical part front portion is pressed against the waist portion of the wearer by the waist portion protecting portion of the protection band for lumbago. Therefore, it is considered that the waist portion protecting portion of the protection band for lumbago firmly adheres to the waist portion of the wearer, and as a result, the waist portion protecting portion of the protection band for lumbago and the cylindrical part rear portion is easily slid relatively in the wearer's stature direction as the wearer makes a bending-and-stretching motion. When the static friction coefficient of the surface of the cylindrical part front portion, which is far from the human body, and the waist portion protecting portion is high, the waist portion protecting portion of the protection band for lumbago more firmly adheres to the waist portion of the wearer, and therefore in this case, the waist portion protecting portion of the protection band for lumbago and the cylindrical part rear portion is easily slid relatively in the wearer's stature direction as the wearer makes a bending-and-stretching motion.

In addition, in the second embodiment as well as the first embodiment, the static friction coefficient of a surface of the cylindrical part front portion, which is close to the human body, i.e. a surface of the cylindrical part front portion on the wearer side, in the bottom wear of the present invention, is preferably 0.2 to 1.0. The underwear worn by the wearer at this surface is in contact with the cylindrical part front portion, but since the static friction coefficient is 1.0 or less, it is possible to suppress a situation in which the bottom wear conforms to the movement of the underwear. On the other hand, when the positional relationship between the underwear and the bottom wear excessively smoothly moves, the wearer may feel uncomfortable. Therefore, the static friction coefficient of this surface is preferably 0.2 or more. The static friction coefficient is more preferably 0.3 or more, still more preferably 0.4 or more because the above-mentioned effect can be further improved. In addition, the static friction coefficient is more preferably 0.8 or less, still more preferably 0.6 or less because the above-described effect can be further improved.

In the second embodiment as well as the first embodiment, the stress of the cylindrical part front portion is 10 N/25 mm or less at 10% elongation in the direction I in the bottom wear of the present invention. Since the stress of the cylindrical part front portion is 10 N/25 mm or less at 10% elongation in the direction I, it is possible to extremely effectively suppress a situation in which the underwear worn by the wearer moves in conformity to the waist-encircling section of the bottom wear as described above. The stress of the cylindrical part front portion is preferably 1 N/25 mm or less at 10% elongation in the direction I because the above-described effect is extremely remarkably exhibited. Here, the direction I of the cylindrical part refers to a direction parallel to the wearer's stature direction or the upper part of the waist-encircling section of the bottom wear. In addition, the lower limit of the stress of the cylindrical part front portion at 10% elongation in the direction I is not particularly limited, but when the cylindrical part front portion is excessively extended, the portion does not completely return to an original length, and strain is apt to remain, so that creases or a feeling of stiffness around the waist may occur. From the viewpoint of the recovery rate from such a condition, the stress of the cylindrical part front portion is preferably 0.2 N/25 mm or more.

In the bottom wear of the first embodiment, the cloth of bottom wear is a cylindrical part rear portion. Preferably, the planar material of the front portion forming the cylindrical part is continuously or intermittently connected to the cloth of bottom wear at the upper part. This connection is established preferably at the upper end in the transverse direction of the cylindrical part, or in the vicinity thereof. The upper part of the planar material means a portion including the upper end of the planar material and a portion situated below the upper end of the planar material. It is desirable that the planar material of the cylindrical part front portion be continuously or intermittently connected to the cloth of bottom wear at the lower part. This connection is established preferably at the lower end in the transverse direction of the cylindrical part, or in the vicinity thereof. The lower part of the planar material means a portion including the lower end of the planar material and a portion situated above the lower end of the planar material. When the cloth of bottom wear is used for about a half of the cylindrical part, it is possible to form a compact cylindrical part as compared to the bottom wear of the second embodiment.

On the other hand, in the bottom wear of the second embodiment, a planar material other than the cloth of bottom wear is used. Preferably, the planar material is continuously or intermittently connected to the cloth of bottom wear at the upper part of the cylindrical part. This connection is established preferably at the upper end in the transverse direction of the cylindrical part, or in the vicinity thereof. The upper part of the planar material means a portion including the upper end of the planar material and a portion situated below the upper end of the planar material. Further, it is desirable that the planar material be continuously or intermittently connected to the back portion of the waist-encircling section in the waist-encircling direction at the lower part of the cylindrical part. This connection is established preferably at the lower end in the transverse direction of the cylindrical part, or in the vicinity thereof. The lower part of the planar material means a portion including the lower end of the planar material and a portion situated above the lower end of the planar material. In this way, the cylindrical part is suspended at the upper part of the cylindrical part, and the lower part of the cylindrical part is partially connected, so that at the time when the protection band for lumbago is inserted into the bottom wear, the silhouette of the appearance from behind is clearly seen.

### EXAMPLES

Hereinafter, the present invention will be described in further detail by way of examples.

### (Measurement methods)

### (Static friction coefficient)

The static friction coefficient of a fabric sample was measured in accordance with Inclination Method in JIS P 8147: 1994 3.2. Three test pieces each having a width of 25 mm and a length of 130 mm were prepared. Next, one of the three test pieces was attached to a weight of 872 g in a slip inclination angle measuring apparatus. On the other hand, a friction target having a width of 120 mm and a length of 200 mm was attached to the slip inclination angle measuring apparatus, and the weight with the test piece was placed in such a manner that the measurement surface of the test piece was in contact with the friction target, and the lengthwise direction of the test piece was coincide with the sliding direction of the slip inclination angle measuring apparatus. The degree of inclination was increased under the condition of an inclination angle of 3°/second, the inclination angle at the time when the weight fell down was read, and the tangent (tan θ) of the inclination angle was defined as a static friction coefficient. For the other two test pieces, the static friction coefficient was measured in the same manner as described above, and an average of the measured values of the static friction coefficients of the three test pieces was defined as a static friction coefficient of the fabric sample. Here, the test piece is sampled in such a manner that the lengthwise direction of the test piece is identical to the direction I of the cylindrical part front portion. In addition, as the friction target, standard cotton (No. 3-1: manufactured by Japanese Standards Association) is used, and the direction of the fabric of this standard cotton may be a warp direction or weft direction.

### (Stress at 10% elongation)

The stress at 10% elongation of the fabric sample was measured using Autograph AG-50kNG manufactured by Shimadzu Corporation. Specifically, five test pieces each having a width of 25 mm and a length of 220 mm were prepared. Next, one of the five test pieces was attached to the above-described measuring equipment at a chuck distance of 100 mm, and pulled at a tensile speed of 300 mm/min until the test piece was broken. The other four test pieces were subjected to the same treatment as described above, and the stress of each of the five test pieces at 10% elongation was measured. The "stress at 10% elongation" mentioned here is an average of the elongation stresses of the five fabrics at an elongation of 10% as measured by the above-described method. Here, the test piece is sampled in such a manner that the lengthwise direction of the test piece is identical to the direction I of the cylindrical part front portion.

### (Hook-and-loop fastener)

The maximum shear strength of the protection band for lumbago at the time of fastening a hook-and-loop fastener was measured in accordance with JIS 3416 using Autograph AG-50kNG manufactured by Shimadzu Corporation. Specifically, five test pieces obtained by cutting both ends of the protection band for lumbago to a length of 150 mm, one of five test pieces for each of a male surface and a female surface were then bonded in such a manner that the test piece overlapped only at a portion where the male surface of the hook-and-loop fastener was placed at the end, and the test piece was attached to the above-described measuring equipment at a chuck distance of 110 mm, and pulled at a tensile speed of 300 mm/min until the fasetened surfaces of the test piece were separated. The other four test pieces were subjected to the same treatment as described above, and the maximum strength was measured for each of the five test pieces, and divided by the value of the fastening area to calculate a maximum strength per area. The "shear strength" mentioned here is an average of the values of the maximum intensity per area of five pieces measured by the above-described method.

### (Evaluation Method)

### (Rising suppression property)

Twenty subjects wore bottom wears of the examples and the comparative examples over the underwear. The protection band for lumbago was inserted into the bottom wear, and then adjusted so that each subject felt a moderate abdominal pressure at the waist portion (pelvic position). Thereafter, the wearer repeatedly made a standing posture and a crouching posture ten times, and grade evaluation was performed for ease of displacement of the protection band for lumbago in the questionnaire on the basis of the following criteria.
1: considerably displaced
2: displaced
3: not so much noticeably displaced
4: hardly displaced
5: not displaced at all

Tables 1 to 3 show the number of subjects who gave any of evaluation points 3 to 5 among 20 subjects. When the number of such subjects is 10 or more, the sample may be rated as having a rising suppression property.

### (Pilling property)

A flat abrasion tester manufactured by DAIEI KAGAKU SEIKI MFG. co., ltd. was used. Two fabrics to be used for the cloth of bottom wear were samples with a width of 40 mm and a length of 250 mm in the longitudinal direction and the transverse direction under a load of 200 g, and flat abrasion treatment was performed (30 times) with the obtained sample and a hook-and-loop (male) to be used for the locking portion of the protection band for lumbago. For each of the samples after the above-described flat abrasion treatment, grade visual evaluation was performed on the basis of the following criteria in accordance with the visual evaluation method in a pilling test as specified in JIS L1076 (2012).
1: dense surface fluffing and/or gross considerable pilling
2: evident surface fluffing and/or evident pilling
3: moderate surface fluffing and/or moderate pilling
4: slight surface fluffing and/or partially formed pill
5: no change).
Here, when the grade was intermediate between two grades, an intermediate grade was given. For example, when "dense surface luffing" (corresponding to evaluation point 1) and "evident fluffing" (corresponding to evaluation point 2) are observed, a grade 1-2 is given. In addition, the "pilling property" refers to one with the lowest numerical value among the grade evaluation results for two samples in the longitudinal and lateral directions.

### (Examples)

### (Example 1)

Polyethylene terephthalate yarns of 167 dtex and spun yarns with 65% of polyester and 35% of cotton were used as warp threads, and polyethylene terephthalate yarns of 167 dtex were used as weft threads to weave a warp double twill fabric at a warp density of 154 threads/2.54 cm and a weft density of 64 threads/2.54 cm, thereby obtaining a cloth A for cloth of bottom wear with 90% of polyester and 10% of cotton.

Subsequently, nylon yarns of 78 dtex and polyurethane elastic fiber of 310 dtex were used to knit a fabric, thereby obtaining a power net cloth with 82% of nylon and 18% of polyurethane (hereinafter, the power net cloth is referred to as a "cloth D").

The cloth A for cloth of bottom wear bottom was used as a cloth of bottom wear to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part front portion (planar material) is the cloth D. In addition, a protection band for lumbago with a configuration as shown in Figs. 4 and 5 was prepared, Mold Magic (registered trademark) (manufactured by Kuraray Fastening Co., Ltd.) of extrusion-molded type was used as a hook-and-loop male surface for the locking portion, and Ring Fastener (manufacture by Seiho Co., Ltd.) was used as a female surface. The protection band for lumbago was defined as a protection band for lumbago 1. The protection band for lumbago 1 was attached to the cylindrical part of the bottom wear as shown in Fig. 6, and the rising suppression property was evaluated. Table 1 shows the physical properties of the fabric forming the cylindrical part and the evaluation results of the bottom wear.

### (Example 2)

Nylon yarns of 44 dtex and polyurethane elastic fiber of 44 dtex were used to knit a tricot, thereby obtaining a tricot cloth with 70% of nylon and 30% of polyurethane (hereinafter, the tricot cloth is referred to as a "cloth E"). The same cloth A as used for the bottom wear in Example 1, and the cloth E were used to prepare a bottom wear having an appearance as shown in Figs. 1 and 2 and an inner part as shown in Fig. 3. That is, the fabric of the cylindrical part rear portion in Fig. 3 is a bottom wear cloth on the back portion in the waist-encircling section of the cloth of bottom wear formed of the cloth A, and the fabric of the cylindrical part rear portion is the cloth E. The protection band for lumbago 1 shown in Figs. 4 and 5 was attached to the cylindrical part of the bottom wear as in Fig. 6, and the rising suppression property was evaluated. Table 1 shows the physical properties of the fabrics forming the cylindrical part and the evaluation results of the bottom wear.

### (Example 3)

PTFE fiber of 3.3 dtex was used as warp threads to prepare a twill fabric with 100% of PTFE (areal weight: 522 g/m²), and the fabric was defined as a cloth B for cloth of bottom wear. The cloth B was used as the bottom wear cloth on the back portion in the waist-encircling section of the cloth of bottom wear, and the cloth A was used for other portions of the cloth of bottom wear to prepare a cloth of bottom wear. Further, the same cloth A as used in Example 1 was used to prepare a bottom wear having an appearance as shown in Figs. 1 and 2 and an inner part as shown in Fig. 3. The fabric of the cylindrical part rear portion in Fig. 3 is the cloth B, and the fabric of the cylindrical part front portion (planar material) is the same cloth D as in Example 1. The protection band for lumbago 1 shown in Figs. 4 and 5 was attached to the cylindrical part of the bottom wear as in Fig. 6, and the rising suppression property was evaluated. Table 1 shows the physical properties of the fabrics forming the cylindrical part and the evaluation results of the bottom wear.

### (Example 4)

PPT composite fiber of 84 dtex and spun yarns with 100% of cotton were used to knit a tricot at 28 G, thereby obtaining a tricot cloth (hereinafter, the tricot cloth is referred to as a "cloth F"). A cloth of bottom wear was prepared in the same manner as in Example 1 using the same cloth A as used in Example 1, and further, the cloth F was used to prepare a bottom wear having an appearance as shown in Figs. 1 and 2 and an inner part as shown in Fig. 3. The fabric of the cylindrical part rear portion in Fig. 3 is the cloth A, and the fabric of the cylindrical part front portion (planar material) is the cloth F described above. The protection band for lumbago 1 shown in Figs. 4 and 5 was attached to the cylindrical part of the bottom wear as in Fig. 6, and the rising suppression property was evaluated. Table 1 shows the physical properties of the fabrics forming the cylindrical part and the evaluation results of the bottom wear.

### (Example 5)

A knit cloth with 100% of cotton, which was subjected to silicon dot processing with a diameter of 1 mm at intervals of 1 mm (the static friction coefficient of a surface subjected to silicon dot processing is 0.91) was defined as a cloth C. The cloth C was used as the bottom wear cloth on the back portion in the waist-encircling section of the cloth of bottom wear, and the cloth A was used for other portions of the cloth of bottom wear to prepare a cloth of bottom wear. Further, the same cloth D as used in Example 1 was used to prepare a bottom wear having an appearance as shown in Figs. 1 and 2 and an inner part as shown in Fig. 3. The fabric of the cylindrical part rear portion in Fig. 3 is the cloth C, and the fabric of the cylindrical part front portion (planar material) is the same cloth D as in Example 1. The protection band for lumbago 1 shown in Figs. 4 and 5 was attached to the cylindrical part of the bottom wear as in Fig. 6, and the rising suppression property was evaluated. Table 2 shows the physical properties of the fabrics forming the cylindrical part and the evaluation results of the bottom wear.

### (Example 6)

A cloth of bottom wear was prepared using the same cloth A as used in Example 1, and further, the same cloth D as used in Example 1 was used to prepare a cylindrical part, thereby preparing a bottom wear having an appearance as shown in Figs. 8 and 2 and an inner part as shown in Fig. 9. The cylindrical part was formed in the following manner: the same cloth D as in Example 1 was folded back at the lower part thereof and stitched and fixed to the cloth of bottom wear as in Fig. 9, the upper part thereof was stitched and fixed over the entire periphery of the back portion of the waist-encircling section, and the lower part thereof was partially stitched and fixed to the waist-encircling section. The fabric of the cylindrical part rear portion in Fig. 9 is the cloth D, and the fabric of the cylindrical part front portion (planar material) is also the cloth D. The protection band for lumbago 1 shown in Figs. 4 and 5 was attached to the cylindrical part of the bottom wear as in Fig. 6, and the rising suppression property was evaluated. Table 2 shows the physical properties of the fabrics forming the cylindrical part and the evaluation results of the bottom wear.

### (Example 7)

In a 30 gauge double tricot knitting machine, polyurethane elastic yarns of 33 decitex were prepared for a front reed, a nylon original yarns of 22 dtex/7 filaments were prepared for a back reed, and a warp knitted fabric having knitted structures at both sides and an elastic yarn content of 53% was knitted in the following structure.
Front reed: 1-0/1-2/2-3/2-1//
Back reed: 2-3/1-1/1-0/2-2//
Next, the obtained warp knitted fabric was scoured. In this way, a tricot cloth having different static friction coefficients on the front and the back (hereinafter, this tricot cloth is referred to as a cloth G). In the tricot cloth, a knit loop was formed with only an elastic yarn to form a warp knitted fabric on one surface of the double knitted structure, a knit loop was formed by use of an elastic yarn and an inelastic yarn in combination to form a warp knitted fabric on the other surface, and the surface and the back surface had static friction coefficients of 0.60 and 0.39, respectively.

A cloth of bottom wear was prepared using the same cloth A as used in Example 1, and further, the cloth G was used to prepare a bottom wear having an appearance as shown in Figs. 8 and 2 and an inner part as shown in Fig. 9. The fabric G was folded back to obtain a member serving as a cylindrical part. The two end portions in the vertical direction of the cloth G and the back portion of the waist-encircling section circumference were continuously stitched and bonded. In addition, the folded-back portion of the cloth G and the back portion of the waist-encircling section were intermittently stitched and bonded. As a result, a cylindrical part was obtained. The former bonding part is situated above the latter bonding part in the bottom wear. In accordance with the characteristics of the cloth, a surface of the cloth G, which has a static friction coefficient of 0.39, is disposed on the inner surface of the cylindrical part rear portion, and a surface of the cloth G, which has a static friction coefficient of 0.60, is disposed on the outer surface of the cylindrical part front portion. The protection band for lumbago 1 shown in Figs. 4 and 5 was attached to the cylindrical part of the bottom wear as in Fig. 6, and the rising suppression property was evaluated. Table 2 shows the physical properties of the fabrics forming the cylindrical part and the evaluation results of the bottom wear.

### (Example 8)

A cloth of bottom wear was prepared using the same cloth A as used in Example 1, and further, a cylindrical part was prepared in the same manner as in Example 7 using the same cloth G as used for the bottom wear in Example 7. However, the cloth is reversed in the front and the back, and in accordance with the characteristics of the cloth, a surface of the cloth C, which has a static friction coefficient of 0.60, is disposed on the inner surface of the cylindrical part rear portion, and a surface of the cloth G, which has a static friction coefficient of 0.39, is disposed on the outer surface of the cylindrical part front portion. The protection band for lumbago 1 shown in Figs. 4 and 5 was attached to the cylindrical part of the bottom wear as in Fig. 6, and the rising suppression property was evaluated. Table 2 shows the physical properties of the fabrics forming the cylindrical part and the evaluation results of the bottom wear.

### (Example 9)

A cloth of bottom wear was prepared using the same cloth A as used in Example 1, and further, the cloth D used in Example 1 was used to prepare a cylindrical part in the same manner as in Example 7, thereby preparing a bottom wear having an appearance as shown in Figs. 8 and 2 and an inner part as shown in Fig. 9. The fabric of the cylindrical part rear portion is the cloth D, and the fabric of the cylindrical part front portion (planar material) is also the cloth D. In addition, a protection band for lumbago with a configuration as shown in Figs. 4 and 5 was prepared, 1QELSX-50J (manufactured by YKK Corporation) of extrusion-molded type was used as a hook-and-loop male surface for the locking portion, and a 2WAY raised cloth was used as a female surface. The protection band for lumbago was defined as a protection band for lumbago 2. The protection band for lumbago 2 was attached to the cylindrical part of the bottom wear as in Fig. 6, and the rising suppression property was evaluated. Table 2 shows the physical properties of the fabrics forming the cylindrical part and the evaluation results of the bottom wear.

### (Example 10)

A cloth of bottom wear was prepared using the same cloth A as used in Example 1, and further, the cloth D used in Example 1 was used to prepare a cylindrical part in the same manner as in Example 7, thereby preparing a bottom wear having an appearance as shown in Figs. 8 and 2 and an inner part as shown in Fig. 9. The fabric of the cylindrical part rear portion is the cloth D, and the fabric of the cylindrical part front portion (planar material) is also the cloth D. In addition, a protection band for lumbago with a configuration as shown in Figs. 4 and 5 was prepared, 1QSK (manufactured by YKK Corporation) of woven fabric tape type was used as a hook-and-loop male surface for the locking portion, and a 2WAY raised cloth was used as a female surface. The protection band for lumbago was defined as a protection band for lumbago 3. The protection band for lumbago 3 was attached to the cylindrical part of the bottom wear as in Fig. 6, and the rising suppression property was evaluated. Table 2 shows the physical properties of the fabrics of the cylindrical parts and the evaluation results of the bottom wear.

### (Comparative Examples)

### (Comparative Example 1)

The same cloth A as used in Example 1 was used to prepare a bottom wear according to the prior art, which had no cylindrical part. The wearer was made to wear the protection band for lumbago 1 with the configuration shown in Figs. 4 and 5, and further wear the bottom wear over the protection band for lumbago, and the rising suppression property was evaluated. Table 3 shows the physical properties of the fabrics of the cylindrical parts and the evaluation results of the bottom wear.

### (Comparative Example 2)

A cloth of bottom wear was prepared using the same cloth A as used in Example 1, and further, the cloth A was used as the planar material of the cylindrical part front portion to prepare a bottom wear having an appearance as shown in Figs. 1 and 2 and an inner part as shown in Fig. 3. The fabric of the cylindrical part rear portion in Fig. 3 is the cloth A, and the fabric of the cylindrical part front portion (planar material) is the cloth A described above. The cloth A had a stress of 12 N at 10% elongation. The protection band for lumbago 1 was attached to the cylindrical part of the bottom wear as in Fig. 6, and the rising suppression property was evaluated. Table 3 shows the physical properties of the fabrics of the cylindrical parts and the evaluation results of the bottom wear.

### (Comparative Example 3)

Three anti-slipping tapes having a length of 14 cm and a width of 25 mm and having two high friction bands continuously disposed from one end portion to the other end portion in the longitudinal direction (800 A manufactured by Ando Tape Co., Ltd.; surface to which a large number of elastic yarns are exposed, has a static friction coefficient of 1.07) were prepared. To the back portion of the waist-encircling section of the same cloth of bottom wear as used in Example 1, these anti-slipping tapes were stitched and bonded at total three positions: the central part of the back portion of the waist-encircling section and portions at a distance of 8 cm on the left and the right from the central part. The longitudinal direction of the anti-slipping tape was made substantially parallel to the vertical direction. This cloth of bottom wear and the same cloth D as used in Example 1 were used to prepare a bottom wear having an appearance as shown in Figs. 1 and 2 and an inner part as shown in Fig. 3. The fabric of the cylindrical part rear portion in Fig. 3 is the cloth A to which three anti-slipping tapes are stitched and bonded, and the fabric of the cylindrical part front portion (planar material) is the same cloth D as in Example 1. The protection band for lumbago 1 shown in Figs. 4 and 5 was attached to the cylindrical part of the bottom wear as in Fig. 6, and the rising suppression property was evaluated. Table 3 shows the physical properties of the fabrics forming the cylindrical part and the evaluation results of the bottom wear.

### (Comparative Example 4)

A cloth of bottom wear was prepared using the cloth A used in Example 1, and the same cloth D as used in Example 1 was used as a planar material of a cylindrical part front portion. In addition, on the surface of a portion (cloth D) of the cylindrical part front portion, which was close to the human body, the anti-slipping tape used in Comparative Example 3 was stitched and bonded at total three positions: the central part of the cloth D (planar material) and portions at a distance of 8 cm on the left and the right from the central part, in such a manner that the longitudinal direction of the anti-slipping tape was substantially parallel to the direction I. Here, a bottom wear having an appearance as shown in Figs. 1 and 2 and an inner part as shown in Fig. 3 was prepared in such a manner that a surface, to which a large number of elastic yarns on the anti-slipping tape were exposed, was inside the bottom wear. The fabric of the cylindrical part rear portion in Fig. 3 is the cloth A, and the fabric of the cylindrical part front portion (planar material), to which three anti-slipping tapes are stitched and bonded, is the cloth D. The protection band for lumbago 1 shown in Figs. 4 and 5 was attached to the cylindrical part of the bottom wear as in Fig. 6, and the rising suppression property was evaluated. Table 3 shows the physical properties of the fabrics forming the cylindrical part and the evaluation results of the bottom wear.

**[Table 1]**

| | | | | Example 1 | Example 2 | Example 3 | Example 4 |
|---|---|---|---|---|---|---|---|
| Bottom wear | Configuration | Cloth of bottom wear | | Cloth A | Cloth A | Cloth B | Cloth A |
| | | Configuration of cylindrical part | | Cylindrical part in first embodiment | Cylindrical part in first embodiment | Cylindrical part in first embodiment | Cylindrical part in first embodiment |
| | | Cylindrical part rear portion | Cloth | Cloth A | Cloth A | Cloth B | Cloth A |
| | | | Static friction coefficient of inner surface | 0,46 | 0,46 | 0,27 | 0,46 |
| | | Cylindrical part front portion | Cloth | Cloth D | Cloth E | Cloth D | Cloth F |
| | | | Stress at 10% elongation in direction I (N/25 mm) | 0,50 | 0,65 | 0,50 | 10 |
| | | | Static friction coefficient of surface of portion close to human body | 0,53 | 0,49 | 0,53 | 0,50 |
| Belt | Configuration | Belt type | | Protection band for lumbago 1 | Protection band for lumbago 1 | Protection band for lumbago 1 | Protection band for lumbago 1 |
| | | Locking portion: | Shear strength (N/cm²) | 11,68 | 11,68 | 11,68 | 11,68 |
| Suppression of rising | | | | 16 | 13 | 17 | 10 |
| Pilling property | | | | 4 | 4 | 4 | 4 |

**[Table 2]**

| | | | | Example 5 | Example 6 | Example 7 | Example 8 | Example 9 | Example 10 |
|---|---|---|---|---|---|---|---|---|---|
| Bottom wear | Configuration | Cloth of bottom wear | | Cloth C | Cloth A | Cloth A | Cloth A | Cloth A | Cloth A |
| | | Configuration of cylindrical part | | Cylindrical part in first embodiment | Cylindrical part in second embodiment | Cylindrical part in second embodiment | Cylindrical part in second embodiment | Cylindrical part in second embodiment | Cylindrical part in second embodiment |
| | | Cylindrical part rear portion | Cloth | Cloth C | Cloth A | Cloth G | Cloth G | Cloth D | Cloth D |
| | | | Static friction coefficient of inner surface | 0,91 | 0,53 | 0,39 | 0,60 | 0,53 | 0,53 |
| | | Cylindrical part front portion | Cloth | Cloth D | Cloth D | Cloth G | Cloth G | Cloth D | Cloth D |
| | | | Stress at 10% elongation in direction I (N/25 mm) | 0,50 | 0,50 | 0,65 | 0,65 | 0,5 | 0,5 |
| | | | Static friction coefficient of surface of portion close to human body | 0,53 | 0,53 | 0,60 | 0,39 | 0,53 | 0,53 |
| Belt | Configuration | Belt type | | Protection band for lumbago 1 | Protection band for lumbago 1 | Protection band for lumbago 1 | Protection band for lumbago 1 | Protection band for lumbago 2 | Protection band for lumbago 3 |
| | | Locking portion: | Shear strength (N/cm²) | 11,68 | 11,68 | 11,68 | 11,68 | 6,18 | 2,08 |
| Suppression of rising | | | | 10 | 17 | 11 | 12 | 12 | 12 |
| Pilling property | | | | 4 | 4 | 4 | 4 | 4 | 2 |

**[Table 3]**

| | | | | Comparative Example 1 | Comparative Example 2 | Comparative Example 3 | Comparative Example 4 |
|---|---|---|---|---|---|---|---|
| Bottom wear | Configuration | Cloth of bottom wear | | Cloth A | Cloth A | Cloth A | Cloth A |
| | | Configuration of cylindrical part | | No cylindrical body | Cylindrical part in first embodiment | Cylindrical part in first embodiment | Cylindrical part in first embodiment |
| | | Cylindrical part rear portion | Cloth | - | Cloth A | Cloth A to which three anti-slipping tapes are stitched and bonded | Cloth A |
| | | | Static friction coefficient of inner surface | - | 0,46 | 1,07 | 0,46 |
| | | Cylindrical part front portion | Cloth | - | Cloth A | Cloth D | Cloth D to which three anti-slipping tapes are stitched and bonded |
| | | | Stress at 10% elongation in direction I (N/25 mm) | - | 12,0 | 0,5 | 15 |
| | | | Static friction coefficient of surface of portion close to human body | - | 0,46 | 0,53 | 1,07 |
| Belt | Configuration | Belt type | | Protection band for lumbago 1 | Protection band for lumbago 1 | Protection band for lumbago 1 | Protection band for lumbago 1 |
| | | Locking portion: | Shear strength (N/cm²) | 11,68 | 11,68 | 11,68 | 11,68 |
| Suppression of rising | | | | 1 | 5 | 7 | 5 |
| Pilling property | | | | 4 | 4 | 4 | 4 |

### DESCRIPTION OF REFERENCE SIGNS

1: Bottom wear
2: Waist-encircling section
3: Cylindrical part
4: Waist-encircling direction
5: Bottom wear cloth on back portion in waist-encircling section
6: Cylindrical part front portion
7: Locking portion
8: Locking portion
9: Protection band for lumbago
10: Waist protecting portion
11: Belt portion
12: First fixing portion
13: Second fixing portion
14: Bone
15: Cylindrical part front portion
15': Cylindrical part rear portion
16: Locking portion
17: Locking portion
18: Back portion
19: Front
20: Rear
21: Space for placement of protection band for lumbago
22: Inner surface of cylindrical part rear portion
23: Outer surface of cylindrical part front portion
24: Cloth of bottom wear
I: Transverse direction of cylindrical part

## Claims

1. A bottom wear comprising a waist-encircling section, wherein
a cylindrical part including a cloth of bottom wear and a planar material different from the cloth, or a cylindrical part including a planar material different from the cloth of bottom wear is present at a back portion of the waist-encircling section and inside the bottom wear,
the cylindrical part is disposed in such a manner that the axis direction of the cylindrical part is substantially parallel to the waist-encircling direction of the waist-encircling section,
the static friction coefficient of the inner surface of a rear portion of the cylindrical part is 0.2 to 1.0, and
the stress of a front portion of the cylindrical part at 10% elongation in the transverse direction of the cylindrical part is 10 N/25 mm or less.

2. The bottom wear according to claim 1, wherein the static friction coefficient of the surface of a part of the front portion of the cylindrical part, which is close to a human body, is 0.2 to 1.0.

3. The bottom wear according to claim 1 or 2, wherein the cylindrical part includes a cloth of bottom wear and a planar material different from the cloth, and
the upper part and the lower part of the planar material are each continuously or intermittently connected to the cloth of bottom wear.

4. The bottom wear according to claim 1 or 2, wherein the cylindrical part includes a planar material different from the cloth of bottom wear, and
the upper part and the lower part of the cylindrical part are each continuously or intermittently connected to the cloth of bottom wear.

5. The bottom wear according to any one of claims 1 to 4, wherein the planar material is a fabric.

6. A bottom wear with a band which includes the bottom wear set forth in any one of claims 1 to 5, and a protection band for lumbago.
